# EUROPÄISCHE PATENTSCHRIFT

(11) **EP 0 804 245 B1**
(45) Veröffentlichungstag und Bekanntmachung des Hinweises auf die Patenterteilung: **26.05.1999**
(21) Anmeldenummer: 95916664.6
(22) Anmeldetag: 15.04.1995
(51) Int. Cl.: A61K 47/42, A61K 9/00, A61K 9/22, A61K 9/26, A61K 9/70

(54) **KOLLAGENZUBEREITUNG ZUR GESTEUERTEN ABGABE VON WIRKSTOFFEN**
COLLAGEN PREPARATION FOR THE CONTROLLED RELEASE OF ACTIVE SUBSTANCES
PREPARATION DE COLLAGENE POUR LA LIBERATION CONTROLEE DE PRINCIPES ACTIFS

(30) Priorität: 27.04.1994 DE 4414755
(43) Veröffentlichungstag der Anmeldung: 05.11.1997
(73) Patentinhaber: LTS LOHMANN Therapie-Systeme GmbH, 56567 Neuwied (DE)
(72) Erfinder: ROREGER, Michael, D-56566 Neuwied (DE)
(74) Vertreter: Flaccus, Rolf-Dieter, Dr.
(86) Internationale Anmeldenummer: EP9501428
(87) Internationale Veröffentlichungsnummer: WO9528964

(56) Entgegenhaltungen:
- EP-A- 0 224 453
- EP-A- 0 518 697
- EP-A- 0 567 234
- AT-B- 71 305
- DE-A- 2 719 770
- DE-A- 2 843 963
- DE-A- 3 429 038
- US-A- 4 619 913
- US-A- 4 774 091
- US-A- 5 024 841
- US-A- 5 206 028

## Beschreibung

Zur Herstellung von Darreichungsformen für pharmazeutische oder kosmetische Wirkstoffe werden eine Reihe von Polymeren eingesetzt, die der jeweiligen Darreichungsform in Abhängigkeit vom Anwendungsort die gewünschten Eigenschaften verleihen sollen. Wundbehandlungsmittel und implantierbare Materialien sollten an die Oberfläche des jeweiligen Applikationsorts angepaßt werden und Körperzellen wie z.B. Keratinocyten, Fibroblasten oder Endothelzellen in ihrer Funktion und Aktivität nicht behindern. Das Spektrum verwendbarer Polymere beschränkt sich in diesen Fällen daher auf solche, die bei Kontakt mit Bindegewebe eine ausgezeichnete Verträglichkeit aufweisen und vorzugsweise biologisch abbaubar sind.

Unter den dabei in Betracht kommenden Polieren nimmt Kollagen, das Hauptprotein des Bindegewebes, seit geraumer Zeit eine Sonderstellung ein aufgrund der biologischen Verträglichkeit und der Abbaubarkeit daraus hergestellter Darreichungsformen. Zum überwiegenden Teil werden solche Kollagenpräparationen ohne weitere Zusätze als Wundabdeckungen eingesetzt. Es hat allerdings auch nicht an Versuchen gefehlt, Kollagenmatrices der unterschiedlichsten Formen mit biologisch aktiven Substanzen zu beladen und die Freisetzung solcher Substanzen, die in der Regel der Auflösung und/oder dem enzymatischen Abbau der Kollagen-Trägermatrix folgt, durch Art, Struktur und Zusammensetzung der Matrix zu beeinflussen.

Zur Herstellung von Kollagenmatrices, wie sie z.B. in US 5 024 841, US 4 789 663, US 4 774 091, US 4 563 350, US 5 246 457 oder EP 429 438 beschrieben sind, werden in der Regel Lösungen von telopeptidfreiem, säurelöslichem Kollagen eingesetzt, aus denen mittels Dialyse, pH-Wert-Verschiebungen oder anderen Verfahren rekonstituierte Fibrillen gewonnen werden, die dann mittels unterschiedlicher Verfahren vorzugsweise zu porösen Matrices verarbeitet werden. Diese Zubereitungen können zwar eine ausgezeichnete Biokompatibilität aufweisen, da sie aus reinem Kollagen bestehen, haben aber den Nachteil, daß sie, bedingt durch die Verwendung löslichen Kollagens, nur in begrenztem Umfang eine Verzögerung der Wirkstofffreisetzung ermöglichen.

Eine solche Freisetzungsverzögerung kann durch Herabsetzung der Löslichkeit der Kollagenmatrix durch Einsatz von Vernetzungs- oder Bindemitteln, wie z.B. in US 4 409 332, DE 2 843 963, WO 93/00890 oder WO 85/04413 beschrieben, durch Einsatz von Verlackungsmitteln, wie in DE 38 41 397 beschrieben, oder durch Kombination von wasserlöslichem Kollagen mit anderen Polymeren, vorzugsweise natürlichen anionischen Polymeren oder deren Derivaten, erreicht werden. In jedem der vorbeschriebenen Fälle müssen allerdings, bedingt durch die zusätzlichen Komponenten, auch zusätzliche toxikologische Risiken in Kauf genommen werden, die insbesondere bei Verwendung der bekannten Vernetzungsmittel für Kollagen nicht zu unterschätzen sind.

Eine andere Möglichkeit zur Verzögerung der Wirkstofffreisetzung wird in EP 518 697 beschrieben. Dabei werden aus wasserlöslichem und/oder wasserunlöslichem Kollagen Laminate hergestellt, die aus einer oder mehreren wirkstoffhaltigen Reservoirschichten und einer die Wirkstoffabgabe verzögernden Schicht bestehen. Solche Laminate könnten im Vergleich zu den vorgenannten Zubereitungen zwar eine Verzögerung der Freisetzung unter Minimierung der toxikologischen Risiken ermöglichen, sie haben aber den Nachteil, daß sie extrem aufwendig in der Herstellung sind und daß ein Aneinanderhaften der Schichten nur mit "feuchten" Filmen erreicht werden kann. Trockene Filme, wie sie für zersetzungsanfällige Wirkstoffe unbedingt notwendig sind, können z.B. nicht zu derartigen Laminaten zusammengefügt werden.

Eine Verzögerung der Wirkstofffreisetzung ist allerdings auch nicht in jedem Fall erwünscht. EP 224 453 beschreibt daher eine Kollagenmatrix, die zum überwiegenden Teil aus wasserunlöslichem, retikuliertem Kollagen besteht und zusätzlich wasserlösliches, nicht retikuliertes Kollagen enthält. In kosmetischen Zubereitungen, z.B. Gesichtsmasken, fungiert dieses lösliche Kollagen als aktive Substanz, die nach der Applikation durch die natürliche Hautfeuchtigkeit oder durch extreme Befeuchtung der Zubereitung herausgelöst wird und auf der Haut zur Wirkung gelangt. Für pharmazeutische Zwecke können in die Zubereitung Wirkstoffe eingearbeitet werden, die dann nach der Applikation zusammen mit dem löslichen Kollagen sehr schnell aus der Kollagenmatrix herausgelöst und freigesetzt werden. Eine derartige Kollagenzubereitung kann dann nützlich sein, wenn eine schnelle Freisetzung erwünscht und sinnvoll ist.

Mechanismen zur Verzögerung oder Beschleunigung der Wirkstofffreisetzung aus Kollagenzubereitungen sind also in vielfältiger Weise beschrieben. Allerdings bietet jede dieser Kollagenzubereitungen nach dem Stand der Technik aufgrund der fest vorgegebenen Zusammensetzung nur eine Möglichkeit der Freisetzungsbeeinflussung und damit ein vorgegebenes Freisetzungsprofil, das jeweils auf eine spezifische Problemlösung, z.B. einen bestimmten Wirkstoff oder eine bestimmte Wirkstoffgruppe, ein bestimmtes Therapieprinzip oder eine bestimmte Erkrankung, zugeschnitten ist. Mit keiner der Kollagenzubereitungen nach dem Stand der Technik kann eine gezielte und dabei vielfältige Steuerung der Wirkstofffreisetzung erreicht werden, d.h. eine variable und individuelle Anpassung der Freisetzungskinetik von Wirkstoffen an jeweils unterschiedliche Problemstellungen, wobei Faktoren wie unterschiedliche Wirkstoffeigenschaften sowie Unterschiede in Wirkungseintritt und Wirkungsdauer jeweils adäquat berücksichtigt werden können

Aufgabe der vorliegenden Erfindung war es daher, eine Kollagenzubereitung zu finden, die nicht nur für einen bestimmten Wirkstoff, eine bestimmte Wirkstoffkombination oder ein bestimmtes Freisetzungsprofil geeignet ist, sondern in einem breiten Rahmen bei unterschiedlichsten Verwendungen eine sichere und an der jeweiligen Problemstellung orientierte Steuerung der Wirkstofffreisetzung ermöglicht.

Überraschenderweise wurde die Lösung der Aufgabe in einer Kollagenzubereitung zur gesteuerten Freisetzung von Wirkstoffen gefunden, die Mischungen säureunlöslicher Kollagene mit unterschiedlichen Molekulargewichtsverteilungen aufweist.

Eine Ausgestaltung sieht vor, daß die Kollagenzubereitung unterschiedliche Wirkstoffe enthält. Sie kann weiterhin Hilfsstoffe wie Viskositätsregulierungsmittel, Bindemittel, Feuchthaltemittel, Weichmacher, Penetrationsbeschleuniger, Konservierungsmittel, Desinfektionsmittel, pH-Regulatoren, Antioxidantien, Wirkstoffstabilisatoren, Öle, Fette, Wachse, Emulsionstabilisatoren, Duftstoffe, Farbstoffe und/oder inerte Füllstoffe enthalten.
Eine bevorzugte Ausgestaltung sieht vor, daß das unlösliche Kollagen telopeptid-freies, natives, unvernetztes Typ-1-Kollagen ist. Es kann sich dabei um ein unlösliches Kollagen handeln, welches ein durch alkalischen Aufschluß aus Kalbshaut gewonnenes Produkt ist.
Weiterhin kann die Kollagenzubereitung in Ausführungsformen von Pulvern, Pudern, Mikropartikeln, Fasern, Flocken, Schäumen, Schwämmen, Nadeln, Stäbchen, Tabletten, Gelen, Cremes, einschichtigen Filmen oder Laminaten vorliegen.

Vorteilhaft kann die Kollagenzubereitung zur Erzielung der gewünschten Freisetzungskinetik Kombinationen unterschiedlicher Arzneiformen umfassen.

Bevorzugt ist die Kollagenzubereitung bioadhäsiv.

Ein Verfahren zur Herstellung der Kollagenzubereitung kann Sprühtrocknung, Gefriertrocknung, Beschichten oder Gießen mit anschließender Trocknung, Phasenseparations- und Koazervationsverfahren, Komprimierung oder Abfüllung in Behältnisse umfassen.
Nach dem Verfahren kann weiterhin die Wirkstoffabgabe durch das Mischungsverhältnis von säureunlöslichen Kollagenen mit unterschiedlichen Molekulargewichtverteilungen beeinflußt und/oder gesteuert werden. Weiterhin ist nach dem Verfahren die Wirkstoffabgabe durch Auflösung oder Quellung oder Erosion der Kollagenzubereitung steuerbar. Eine weitere Steuermöglichkeit der Wirkstofffreisetzung ist durch biologischen Abbau der Kollagenzubereitung möglich.
Die Verwendung der erfindungsgemäßen Kollagenzubereitung besteht in der gesteuerten Abgabe von Wirkstoff an Wunden. Die Verwendung kann aber auch auf die gesteuerte Abgabe von Wirkstoff an intakte Haut gerichtet sein. Und schließlich kann die Verwendung der Kollagenzubereitung zum Implantieren oder Injizieren von Wirkstoff in einen lebenden Organismus dienen.

Kollagenzubereitungen zur Wirkstoffabgabe nach dem Stand der Technik werden in der Regel aus säurelöslichem Kollagen hergestellt, das heißt Kollagen, das in verdünnten Säuren bei pH 2 klar gelöst ist. Dieses säurelösliche Kollagen kann mit unterschiedlichsten Verfahren aus einer Reihe von Geweben tierischen und pflanzlichen Ursprungs isoliert werden.

Demgegenüber handelt es sich bei dem erfindungsgemäß verwendeten Kollagen um säureunlösliches Kollagen, das, wenn es in wäßriger Dispersion vorliegt, auch durch Zugabe von konzentrierter Essigsäure nicht in Lösung zu bringen ist. Dieses unlösliche Kollagen ist vorzugsweise natives Kollagen, das zum überwiegenden Teil als Typ I Kollagen und zu einem geringeren Teil als Typ III Kollagen vorliegt. Die Bezeichnung natives Kollagen bezieht sich auf ein Kollagenmolekül, das eine unveränderte tripelhelikale Tertiärstruktur aufweist.

Das erfindungsgemäß verwendete unlösliche Kollagen kann in Abwandlung von Verfahren, wie sie z.B. in DE-OS 3034273, US 4 021 522 oder DE-OS 27 16 602 beschrieben sind, durch alkalischen Aufschluß aus Biomaterial verschiedenen Ursprungs erhalten werden. Vorzugsweise dient als Ausgangsmaterial 6 Monate alte Kalbshaut. Dadurch wird, im Gegensatz zu den üblicherweise verwendeten Geweben und Körperteilen von Rindern, Schweinen oder Pferden, sichergestellt, daß ein Ausgangsmaterial von definierter, gleichbleibender Qualität zur Verfügung steht, was wiederum die zuverlässige Reproduzierbarkeit der im folgenden beschriebenen und im Beispiel 1 detailliert dargestellten Verfahrensschritte gewährleistet. Die Kalbshaut wird zunächst mechanisch enthaart und entfettet. Danach werden lösliche Kollagene und nichtkollagene lösliche Bestandteile des Bindegewebes extrahiert und verworfen. Im nächsten Schritt wird das Bindegewebe zunächst mit einer wäßrigen Lösung eines Alkalihydroxids, vorzugsweise Natriumhydroxid, und eines Alkalisulfats, vorzugsweise Natriumsulfat, und anschließend mit einer wäßrigen Alkalisulfat-Lösung behandelt zum Verseifen und Herauslösen der Hautfette sowie zur gesteuerten Quellung der Kollagenfasern. Dabei werden auch die endständigen, nichthelikalen Teile der Kollagenmoleküle, die sogenannten Telopeptide, die überwiegend für die antigenen Eigenschaften xenogenen Kollagens verantwortlich sind, abgespalten. Des weiteren wird durch die Behandlung mit Alkalihydroxid/Alkalisulfat- bzw. reiner Alkalisulfat-Lösung in Abhängigkeit von der Konzentration und der Einwirkzeit der Lösungen ein definierter Teil der intermolekularen Kollagenbindungen im Faserverbund des geguollenen Bindegewebes gespalten. Die intramolekularen Bindungen des Kollagens werden nicht angegriffen, so daß die helikale Struktur des Moleküls intakt bleibt. Das so aufgeschlossene Bindegewebe wird in mehreren Stufen mit Wasser und verdünnten Säuren ausgewaschen, gereinigt und neutralisiert, anschließend mechanisch zerkleinert und in Wasser dispergiert.

Der entscheidende Schritt für die Isolierung des erfindungsgemäß verwendeten, säureunlöslichen Kollagens mit unterschiedlichen Molekulargewichtsverteilungen ist die gezielte alkalische Spaltung der inter-molekularen Kollagenbindungen. Läßt man z.B. eine wäßrige Lösung von 9,75 % Natriumhydroxid und 9,2 % Natriumsulfat 48 Stunden auf das gequollene Bindegewebe einwirken, so weist nach dem Dispergieren in Wasser das unlösliche Kollagen bei HPLC-Analyse (Säulensystem Biosil TSK-400 + Biosil TSK-125; Eluent 0,5 m Ammoniumacetatpuffer pH 6.7; Detektor UV 275 nm, Empfindlichkeitsbereich 0,02 AUFS) mit Auswertung gegen Eichprotein-Chromatogramme ein mittleres Molekulargewicht von ca. 420.000 auf. Behandelt man hingegen das gequollene Bindegewebe nur 12 Stunden mit einer wäßrigen Lösung von nur 5% Natriumhydroxid und 9,2% Natriumsulfat, so werden deutlich weniger intermolekulare Bindungen gespalten, und die nach dem Dispergieren in Wasser erhaltenen unlöslichen Molekülaggregate zeigen bei der Analyse ein mittleres Molekulargewicht von ca. 2.500.000. Diese Unterschiede wirken sich zunächst auf das Fließverhalten und damit auf die Verarbeitbarkeit der Dispersionen unlöslichen Kollagens aus. Während eine Dispersion mit 5 % niedermolekularem, unlöslichem Kollagen eine zwar zähe, aber noch freifließende Masse ist, liegt die Fließgrenze einer Dispersion höhermolekularen, unlöslichen Kollagens bei ca. 2,5 %.

Die variablen Faktoren des Aufschlußverfahrens, durch deren Variation jeweils unterschiedliche Molekulargewichtsverteilungen erreicht werden können, sind also Konzentration und Einwirkzeit von Natriumhydroxid. Je höher die Konzentration an Natriumhydroxid und/oder je länger die Einwirkzeit von Natriumhydroxid, desto geringer ist das mittlere Molekulargewicht des isolierten, säureunlöslichen Kollagens, und umgekehrt. Um den Zusammenhang zwischen erzielbarem Molekulargewicht und den genannten Aufschlußbedingungen z.B. in Form einer Kurve darstellen zu können, müßte einer der Einflußfaktoren, also Konzentration oder Einwirkzeit, konstant gehalten werden, während der andere verändert wird. Dies ist zwar grundsätzlich möglich, unter produktions- und betriebstechnischen Gesichtspunkten aber wenig sinnvoll, da z.B. eine freie Variation der Einwirkzeit allein in der Regel nicht möglich ist. Durch sehr lange Einwirkzeiten bei entsprechend niedriger Natriumhydroxid-Konzentration z.B. würden Maschinenlaufzeiten verlängert und Personaleinsatz erhöht, was unter dem Gesichtspunkt der Betriebskosten nicht akzeptabel ist. Andererseits führen sehr kurze Einwirkzeiten, bedingt durch die dadurch notwendige höhere Natriumhydroxid-Konzentration, zu einem erhöhten Verschleiß an Produktionsanlagen, wie z.B. Leitungs- und Filtersystemen, was ebenfalls wegen der damit verbundenen Betriebskostensteigerung nicht umzusetzen ist. Notwendige Natriumhydroxid-Einwirkzeit und -Konzentration müssen daher für jede Problemstellung individuell empirisch so ermittelt werden, daß sie sowohl in Hinsicht auf die Anforderungen an die Arzneiform und insbesondere an die Wirkstofffreisetzungskinetik als auch unter Wirtschaftlichkeitsaspekten ein Optimum darstellen.

Die Unterschiede in der Molekulargewichtsverteilung unlöslichen Kollagens haben einen deutlichen Einfluß auf die Eigenschaften daraus durch Trocknung hergestellter Kollagenzubereitungen. In Beispiel 2 ist dargestellt, daß durch Gefriertrocknung hergestellte Schäume aus unlöslichem Kollagen unterschiedlicher Molekulargewichtsverteilungen in Abhängigkeit vom Mischungsverhältnis sehr unterschiedliche Zerfallseigenschaften zeigen. Während Schäume aus 100 % nieder-molekularem, unlöslichem Kollagen in künstlichem Wundsekret von pH 6,4 bereits nach 45 Minuten vollständig zerfallen sind, sind unter gleichen Bedingungen Schäume aus 100 % höhermolekularem, unlöslichem Kollagen auch nach 10 Tagen noch nicht zerfallen.

Das Beispiel zeigt, daß die Verwendung von hochmolekularen Kollagenaggregaten einen deutlich stabilisierenden Einfluß auf den Schaum hat, was in deutlich verlangsamter Sekretaufnahme und Quellung und in einem sehr stark verzögerten Zerfall zum Ausdruck kommt. Die Formstabilisierung durch den Erhalt eines hohen natürlichen Vernetzungsgrads des Kollagens hat vor allem aus toxikologischer Sicht den entscheidenden Vorteil, daß zusätzliche Manipulationen zur Verfestigung der Struktur, wie z.B. Gerbung und Vernetzung, überflüssig werden. Das Produkt erfährt seine Formstabilisierung nur dadurch, daß die ursprüngliche Quartärstruktur des Kollagens, wie sie in der Haut vorliegt, weitgehend erhalten bleibt.

Die Kollagenschäume, die jeweils nur aus einem einzigen Grundtyp unlöslichen Kollagens hergestellt wurden, zeigen also deutliche Unterschiede, z.B. in Bezug auf Interaktion mit Wundsekret. Allerdings können derartige Mono-Zubereitungen, wie eingangs erläutert, nur in speziellen Einzelfällen dem aus einem bestimmten Anwendungszweck resultierenden Anforderungsprofil an ein Kollagenprodukt gerecht werden. Demgegenüber bietet die vorliegende Erfindung auf zweierlei Weise die Möglichkeit, die Eigenschaften einer Kollagenzubereitung auf gegebene Produkt-Anforderungsprofile jeweils genau zuzuschneiden. Zum einen kann die Molekulargewichtsverteilung des unlöslichen Kollagens durch gezielte Steuerung der Spaltung intermolekularer Bindungen in sehr breitem Rahmen stufenlos variiert werden. Zum anderen können durch Abmischung dieser Variationen unlöslichen Kollagens mit jeweils unterschiedlichen Molekulargewichtsverteilungen die erforderlichen Produkteigenschaften eingestellt werden. Dies wird z.B. deutlich bei Fortführung des bereits genannten Beispiels 2 anhand des Zerfalls gefriergetrockneter Schäume, bei denen vor der Trocknung unlösliches Kollagen mit einem mittleren Molekulargewicht von ca. 420.000 und unlösliches Kollagen mit einem mittleren Molekulargewicht von ca. 2.500.000 in jeweils unterschiedlichen Verhältnissen gemischt wurden. Die Zerfallszeiten zeigen bei unterschiedlichen pH-Werten immer eine stufenweise Abnahme bei stufenweiser Erhöhung des prozentualen Anteils unlöslichen Kollagens mit niedrigerem mittleren Molekulargewicht.

Der exakten Einstellung der Produkteigenschaften an ein therapieoptimales Anforderungsprofil kommt vor allem in der Therapie mit pharmazeutischen Wirkstoffen Bedeutung zu.

Da die erfindungsgemäße Kollagenzubereitung vorzugsweise auf der Haut, auf externen und internen Wunden sowie in internen Körpergeweben und -höhlen nach Implantation oder Injektion Anwendung findet, sind die für die Beladung der Kollagenzubereitung in Betracht kommenden Wirkstoffe vorzugsweise Wirkstoffe zur dermalen und transdermalen Applikation, Wirkstoffe zur Wundbehandlung und Förderung der Wundheilung sowie Wirkstoffe, wie sie üblicherweise mittels Zubereitungen zur Implantation oder Injektion verabreicht werden.

Zur dermalen Behandlung lokaler Hauterkrankungen werden Lokalanästhetika, Lokalantibiotika, Antiseptika, Antimykotika, Antihistaminika und juckreizstillende Arzneistoffe, Keratolytika und ätzende Arzneistoffe, Virustatika, Antiskabiesmittel, Steroide, sowie verschiedene Substanzen zur Behandlung von Akne, Psoriasis oder Lichtdermatosen verwendet. Zu den Wirkstoffen, die intradermal appliziert werden, gehören z.B. steroidale und nichtsteroidale Antirheumatika, durchblutungsfördernde Substanzen oder Vasoprotektoren und -konstriktoren zur Behandlung von Gefäßerkrankungen. Zu den transdermal applizierbaren Wirkstoffen gehören z.B. Neuroleptika, Antidepressiva, Tranquillantien, Hypnotika, Psychostimulantien, Analgetika, Muskelrelaxantien, Antiparkinsonmittel, Ganglienblocker, Sympathomimetika, Alpha-Sympatholytika, Beta-Sympatholytika, Antisympathotonika, Antidiabetika, Koronartherapeutika, Antihypertonika, Antiasthmatika oder Diuretika. Ebenfalls auf der Haut kann die erfindungsgemäße Kollagenzubereitung auch Anwendung finden in kosmetischen Präparaten, z.B. in Form von Schaummasken oder Filmen zur Behandlung von z.B. gealterter Haut, Falten oder unreiner Haut, zur Körperpflege, zur Haarentfernung, zur Verminderung der Schweißabsonderung oder zum Lichtschutz.

Wirkstoffe, die in den erfindungsgemäßen Kollagenzubereitungen auf externen und internen Wunden zur Anwendung kommen, sind vorzugsweise blutstillende Wirkstoffe, unter denen Kollagen selbst eine besondere Rolle spielt, wundreinigende Wirkstoffe wie z.B. Enzyme, Antiseptika, Desinfizientia und Antibiotika sowie wundheilungsfördernde Wirkstoffe, durch die die Granulation angeregt, die Gefäßneubildung induziert oder die Epithelisierung gefördert wird. Unter den wundheilungsfördernden Wirkstoffen gewinnen biologisch aktive Peptide und Proteine, die bereits in sehr geringer Konzentration hohe Aktivitäten entfalten, und zum großen Teil mittels rekombinanter Technologien hergestellt werden, zunehmend an Bedeutung. Zu diesen Substanzen, für die die erfindungsgemäße Kollagenzubereitung ein besonders geeignetes Träger- und Abgabesystem darstellt, gehören sog. Wachstumsfaktoren wie Platelet derived growth factor (PDGF), Epidermal growth factor (EGF), Platelet derived endothelial cell growth factor (PD-ECGF), acidic Fibroblast growth factor (aFGF), basic Fibroblast growth factor (bFGF), Transforming growth factor α (TGF α), Transforming growth factor β (TGF β), Keratinocyte growth factor (KGF), Insulin-like growth factors 1 und 2 (IGF1, IGF2) sowie Tumor necrosis factor (TNF).

Zu den Wirkstoffen, die mittels der erfindungsgemäßen Kollagenzubereitung parenteral verabreicht werden, gehören z.B. Antibiotika, Antiseptika, Anästhetika, Analgetika unterschiedlicher Stärke, Cytostatika, Hormone, Steroide, Cytokine wie z.B. Interleukine, Interferone und koloniestimulierende Faktoren, Hormone releasing und release inhibiting Faktoren, Prostaglandine, Enzyme sowie Wachstumsfaktoren, insbesondere osteoinduktiv wirksame Knochenwachstumsfaktoren.

Eine der größten Herausforderungen für die Entwicklung von wirkstoffhaltigen Zubereitungen ist es, Formulierungen zu finden, die den Wirkstoff so freisetzen, daß ein Wirk- und damit Therapieoptimum erreicht wird. Viele Wirkstoffe, insbesondere die genannten biologisch aktiven Peptide und Proteine, haben im Körper nur eine kurze Halbwertszeit und müssen daher oft mehrmals täglich verabreicht werden.

Daher kommt Wirkstoffträgern, die die Wirkstoffe verzögert oder gar kontrolliert freisetzen, zunehmende Bedeutung zu. Kollagenzubereitungen gemäß der vorliegenden Erfindung bieten die Möglichkeit, mit relativ wenigen Formulierungsgrundbausteinen Trägersysteme zu entwickeln, die sehr flexibel in den Anwendungs- und Gestaltungsmöglichkeiten sind und die sehr genau auf die erforderliche Problemlösung ausgerichtet werden können. Die Mechanismen, die der Freisetzungskinetik von Wirkstoff ihre Charakteristik verleihen, können durch Abmischung unlöslichen Kollagens unterschiedlicher Molekulargewichte und Formgebung der Kollagenzubereitung gezielt gesteuert werden. Durch diese Faktoren werden neben Struktur und Dichte des polymeren Kollagengerüsts auch Anzahl und Verteilung hydrophiler, hydrophober und ionischer Bindungsstellen entlang des Polymergerüsts beeinflußt. Da Wirkstoff nicht nur in Hohlräume einer erfindungsgemäßen Kollagenzubereitung eingeschlossen, sondern auch an die Oberfläche des Kollagengerüsts adsorbiert werden kann, wird die Bindungsstärke zwischen Kollagen und Wirkstoff und somit auch dessen Freisetzungsverhalten maßgeblich durch ionische Beziehungen sowie hydrophile und hydrophobe Wechselwirkungen bestimmt.

Bei der dermalen, intradermalen und transdermalen Applikation von Wirkstoff mit einer erfindungsgemäßen Kollagenzubereitung haben neben Struktur, Dichte und Bindungsaktivität der Kollagenzubereitung auch die Löslichkeit von Wirkstoff in der Zubereitung, Beladungs- und Sättigungsgrad sowie Diffusionsgeschwindigkeit von Wirkstoff innerhalb der Zubereitung Einfluß auf das Freisetzungsverhalten.

Bei Kollagenzubereitungen zur Abgabe von Wirkstoff an externe oder interne Wunden sowie bei Kollagenzubereitungen zur Implantation oder Injektion kommt ein weiterer Faktor hinzu.

Da in den genannten Anwendungsfällen die erfindungsgemäßen Kollagenzubereitungen mit Körperflüssigkeit in Berührung kommen, können, wie in dem bereits erwähnten Beispiel 2 gezeigt, Geschwindigkeit und Umfang der Flüssigkeitsaufnahme durch die Kollagenzubereitung und somit Quellvermögen und Zerfallseigenschaften der Kollagenzubereitung als Freisetzungssteuerungsmöglichkeiten genutzt werden. Neben dem Zerfall der Kollagenzubereitung als steuerndem Mechanismus kommen bezüglich der Freisetzungssteuerung bei Kontakt mit Körperflüssigkeit vor allem das Herauslösen von Wirkstoff aus der Kollagenzubereitung durch Körperflüssigkeit sowie die flüssigkeitsvermittelte Diffusion von Wirkstoff aus dem Kern der Kollagenzubereitung an deren Grenzfläche in Betracht. Darüber hinaus wird die Freisetzung beeinflußt durch biologischen Abbau der Kollagenzubereitung mittels Hydrolyse und enzymatischer Einwirkung bei Kontakt mit Körperflüssigkeit. Anhand von Beispiel 2 ist bereits die große Bandbreite der Wechselwirkung zwischen Flüssigkeit und Zubereitungen aus Abmischungen unlöslichen Kollagens unterschiedlicher Molekulargewichtsverteilungen dargestellt worden. Wie sich diese Wechselwirkungen im Zusammenwirken mit einem oder mehreren der genannten Einflußfaktoren auf die Freisetzungskinetik von Wirkstoff auswirkt, ist anschaulich in den Beispielen 3 und 4 dargestellt. Es wird deutlich, daß das Mischungsverhältnis unlöslichen Kollagens unterschiedlicher Molekulargewichtsverteilungen signifikant die Wirkstofffreisetzungskinetik bestimmt. Dadurch ist es möglich, bei gegebenem Wirkstoff und vorgegebener Soll-Freisetzung pro Zeit die Kollagenzubereitung gezielt an die geforderten Vorgaben anzupassen, um so nach Applikation eine therapieoptimale Wirkstoffdosierung über den vorgesehenen Anwendungszeitraum zu erzielen. Soll bei der in den Beispielen 3 und 4 gewählten Arzneiform, hergestellt aus den in Beispiel 1 beschriebenen Zubereitungen unlöslichen Kollagens mit unterschiedlichen Molekulargewichtsverteilungen, Wirkstoff in einem Anwendungszeitraum von 24 bis 48 Stunden abgegeben werden, so sollte das Mischungsverhältnis von niedermolekularem und hochmolekularem unlöslichem Kollagen nicht kleiner als 3 zu 1 sein. Soll andererseits eine gleichmäßig hinhaltende Wirkstofffreisetzung aus einer vergleichbaren Arzneiform in einem Anwendungszeitraum von 7 bis 14 Tagen erfolgen, so sollte das Mischungsverhältnis von niedermolekularem und hochmolekularem unlöslichem Kollagen nicht größer als 1 zu 3 sein.

In den Beispielen wurden bewußt nur reine Kollagenzubereitungen plus Wirkstoff untersucht, um die Steuerungsmöglichkeiten von Zubereitungen aus Abmischungen unlöslichen Kollagens unterschiedlicher Molekulargewichtsverteilungen frei von Einflüssen sonstiger Hilfsstoffe zu belegen. In der Praxis wird man allerdings ohne zusätzliche wasserlösliche oder wasserdispergierbare Additive kaum auskommen können, da neben den Ansprüchen an die Wirkstofffreisetzung in der Regel, vom Anwendungszweck her kommend, auch Ansprüche an Handhabungseigenschaften und Stabilität einer Wirkstoff-Kollagen-Zubereitung gestellt werden.

Solche Hilfsstoffe können zusätzliche Polymere, die z.B. als Viskositätsregulierungsmittel bei Verwendung flüssiger Zubereitungen oder als Bindemittel bei festen Formen dienen, wie z.B. Cellulosederivate, Stärkederivate, Galaktomannanderivate, Dextrane, pflanzliche Polysaccharide wie Alginate, Pektine, Carrageenan oder Xanthan, Chitosan, Proteine, Glykoproteine, Proteoglykane, Glucosaminoglykane, Polyvinylalkohol, Polyvinylpyrrolidon, Vinylpyrrolidon-Vinylacetat-Mischpolymerisate, Polyethylenglykol, Polyacrylate und Polymethacrylate, Polylactide und Polyglycolide sowie Polyaminosäuren sein.

Als weitere Hilfsstoffe kann die Kollagenzubereitung umfassen
- Feuchthaltemittel wie beispielsweise Glycerin, Sorbitol, Polyethylenglykol, Polypropylenglykol
- Weichmacher wie beispielsweise Citronensäureester, Weinsäureester oder Glycerinester
- Penetrationsbeschleuniger wie beispielsweise Alkylsulfate, Alkylsulfonate, Alkaliseifen, fettsaure Salze von mehrwertigen Metallen, Betaine, Aminoxide, Fettsäureester, Mono-, Di- oder Triglyceride, langkettige Alkohole, Sulfoxide, Nicotinsäureester, Salicylsäure, N-Methylpyrrolidon, 2-Pyrrolidon oder Harnstoff.
- Konservierungsmittel wie beispielsweise p-Cl-m-Kresol, Phenylethylalkohol, Phenoxyethylalkohol, Chlorbutanol, 4-Hydroxybenzoesäuremethylester, 4-Hydroxybenzoesäurepropylester, Benzalkoniumchlorid, Cetylpyridiniumchlorid, Chlorhexidindiacetat oder -digluconat, Ethanol oder Propylenglykol enthalten.
- Desinfektionsmittel wie bspw. Halogene wie Polyvidon-Jod, Halogenverbindungen wie Natriumhypochlorit oder Tosylchlorid-Natrium, Oxidationsmittel wie Wasserstoffperoxid oder Kaliumpermanganat, Arylquecksilberverbindungen wie Phenylmercuriborat oder Merbromin, Alkylquecksilberverbindungen wie Thiomersal, Organozinnverbindungen wie Tri-n-butyl-zinnbenzoat, Silberweißverbindungen wie Silberweißacetyltannat, Alkohole wie Ethanol, n-Propanol oder Isopropanol, Phenole wie Thymol, o-Phenylphenol, 2-Benzyl-4-Chlorphenol, Hexachlorophen oder Hexylresorcin oder organische Stickstoffverbindungen wie 8-Hydroxychinolin, Chlorquinaldol, Clioquinol, Ethacridin, Hexetidin, Chlorhexidin oder Ambazon.
- pH-Regulatoren wie beispielsweise Glycinpuffer, Citratpuffer, Boratpuffer, Phosphatpuffer oder Citronensäure-Phosphat-Puffer.
- Antioxidantien wie bspw. Ascorbinsäure, Ascorbylpalmitat, Tocopherolacetat, Propylgallat, Butylhydroxyanisol oder Butylhydroxytoluol.
- Wirkstoffstabilisatoren wie Mannitol, Glucose, Lactose, Fructose, Saccharose
- Emulgierbare Hilfsstoffe wie Öle, Fette und Wachse
- Duftstoffe, Farbstoffe, Reinigungsstoffe, Körperpflegestoffe
- Emulsionsstabilisatoren wie z.B. nichtionogene Emulgatoren amphotere Emulgatoren, kationaktive Emulgatoren und anionaktive Emulgatoren
- Füllstoffe wie z.B. mikrokristalline Cellulose, Aluminiumoxid, Zinkoxid, Titandioxid, Talkum, Siliciumdioxid, Magnesiumsilikat, Magnesium-Aluminiumsilikat, Kaolin, hydrophobe Stärke, Calciumstearat oder Calciumphosphat.

Hilfsstoffe können z.B. vor dem Arzneiformungsprozeß in Dispersionen unlöslichen Kollagens gelöst, dispergiert oder emulgiert werden. Sie können aber auch nach Abschluß eines der unten dargestellten Primärformungsprozesse der Abmischungen unlöslichen Kollagens erst in späteren Stufen der Arzneiformung in der für die jeweiligen Formungsprozesse üblichen und bekannten Art und Weise eingebracht werden.

Die Ausführungsformen, in denen Abmischungen unlöslichen Kollagens unterschiedlicher Molekulargewichtsverteilungen zur gesteuerten Abgabe von Wirkstoffen zur Anwendung gelangen können, sind ausgesprochen vielfältig und können daher nicht umfassend dargestellt werden.
Diese Ausführungsformen der erfindungsgemäßen Kollagenzubereitung können nach unterschiedlichen, dem Fachmann geläufigen Methoden aus Mischungen von Dispersionen unlöslichen Kollagens mit jeweils unterschiedlichen Molekulargewichtsverteilungen hergestellt werden durch z.B. Sprühtrocknung, Gefriertrockung, Beschichtung oder Gießen mit anschließender Trocknung, Phasenseparations- und Koazervationsverfahren für Partikel oder emulgierte Tröpfchen, Trocknung und Komprimierung sowie einfache Abfüllung in Behälter wie z.B. Tuben und resultieren in z.B. Pulvern, Pudern, Mikropartikeln, Fasern, Flocken, Schäumen, Schwämmen, Nadeln, Stäbchen, Tabletten, Gelen, Cremes, einschichtigen Filmen oder Laminaten. Bevorzugte Ausführungsformen sind sprühgetrocknete Mikropartikel, gefriergetrocknete Schäume und durch Beschichtung hergestellte, gelartige Filme.

Das Einbringen von Wirkstoff in die erfindungsgemäße Kollagenzubereitung kann so erfolgen, daß Wirkstoff in der fertigen Mischung von Dispersionen unlöslichen Kollagens mit unterschiedlicher Molekulargewichtsverteilung vor dem Zubereitungsformungsprozeß gelöst oder dispergiert wird. Soll mehr als ein Wirkstoff eingearbeitet werden, können diese auch vor dem Mischvorgang in den einzelnen Fraktionen unlöslichen Kollagens getrennt voneinander gelöst oder dispergiert werden. Wirkstoff kann aber auch nach erfolgtem Zubereitungsformungsprozeß durch Coating-, Sprüh-, Imprägnierungs-, Tauch- oder andere Adsorptionsverfahren auf bzw. in die Zubereitung gebracht werden.

Die möglichen Ausführungsformen, die entsprechenden Herstellverfahren und die Methoden zum Eintrag von Wirkstoff können aber auch miteinander kombiniert werden zur Erzielung bestimmter Eigenschaften.

So kann die erfindungsgemäße Kollagenzubereitung z.B. eine in Körperflüssigkeit lösliche oder zumindest quellbare Hülle unlöslichen Kollagens mit niedrigem mittleren Molekulargewicht in Schwamm- oder Filmform und eine darin dispergierte mikropartikuläre Zubereitung unlöslichen Kollagens mit hohem mittleren Molekulargewicht umfassen. Bei Einarbeitung nur eines Wirkstoffs dient die äußere Schwamm- oder Filmphase der schnellen Freisetzung von Wirkstoff zur schnellen Erreichung der mindest notwendigen Wirkstoffkonzentration, gefolgt von einer langsameren, gleichmäßigeren Freisetzung von Wirkstoff aus der inneren partikulären Phase zur Aufrechterhaltung der notwendigen Wirkstoffkonzentration über den Applikationszeitraum. Derartige Freisetzungsprofile können auch mit anderen Zubereitungsformen wie z.B. Fasern in Hydrogelen, schwammartigen Öl-in-Wasser-Emulsionen, Komprimatmischungen für implantierbare Tabletten, mehrschichtigen Filmen, Kombinationen von Filmen und Schäumen u.s.w. erreicht werden.

Derartige mehrphasige Zubereitungen können auch verwendet werden, wenn die Freisetzung unterschiedlicher Wirkstoffe zu unterschiedlichen Zeitpunkten mit unterschiedlichen Freisetzungsraten erwünscht ist oder wenn einer oder mehrere Wirkstoffe phasisch, das heißt mit freisetzungsfreien Intervallen freigesetzt werden sollen.
Dabei kann z.B. Wirkstoff A in einer leichtlöslichen, schnell freisetzenden äußeren Phase enthalten sein, während Wirkstoff B in einer schwer löslichen, retardiert und kontrolliert hinhaltend freisetzenden inneren Phase enthalten ist. Wird ein phasisches Freisetzungsprofil für nur einen Wirkstoff gewünscht, so können äußere und innere Phase z.B. mit gleicher Kinetik Wirkstoff freisetzen. Die innere Phase würde in diesem Fall allerdings mit einer wirkstofffreien Schicht unlöslichen Kollagens ummantelt, die nach Auflösung der äußeren Phase oder deren Erschöpfung an Wirkstoff selbst erst quellen oder sich auflösen muß, damit die Wirkstofffreisetzung aus der inneren Phase erfolgen kann. Durch diese Anordnung kann ein Intervall erhalten werden, in dem kein Wirkstoff freigesetzt wird.

Bei Verwendung der erfindungsgemäßen Kollagenzubereitung zur dermalen, interdermalen oder transdermalen Applikation von pharmazeutischen Wirkstoffen oder kosmetischen Aktivsubstanzen werden flächige Ausführungsformen wie Filme, Membranen oder dünne Schwämme bevorzugt. Diese flächigen Ausführungsformen können aus Laminaten bestehen, die z.B. auch wirkstofffreie Sperrschichten, durchlässige Trennschichten, Steuermembranen und Klebschichten umfassen. In oder zwischen die einzelnen Schichten können wiederum Substrukturen wie z.B. Plättchen, Pulver, Mikropartikel oder Öltröpfchen eingebracht sein, um die für einen oder mehrere Wirkstoffe geeignete Freisetzungskinetik zu erreichen. Vorzugsweise werden derartige ein- oder mehrschichtige Kollagenzubereitungen für die genannten Anwendungen z.B. zum Schutz vor Austrocknung oder dem Einwachsen von Keimen nach bekannten Verfahren mit einer Rückschicht und auf der gegenüberliegenden Seite mit einer ablösbaren Schutzschicht versehen, wobei Rückschicht und Schutzschicht aus den dem Fachmann geläufigen Materialien bestehen können, wie sie z.B. bei der Formulierung von Pflastern oder Klebebändern verwendet werden.

In einer bevorzugten Ausführung der erfindungsgemäßen Kollagenzubereitung für die Anwendung auf externen Wunden sowie im Körperinnern ist die Zubereitungsform porös, z.B. schaum- oder schwammartig. Die Größe der Poren und die Struktur der Zubereitung ist so ausgelegt, daß das Einwandern von Zellen wie z.B. Fibroblasten oder Osteoblasten in die Zubereitung möglich ist und den Zellen dabei eine strukturelle Orientierung gegeben wird, die insbesondere auf den dem natürlichen Bindegewebe ähnlichen Ordnungsgrad des Kollagens in der erfindungsgemäßen Zubereitung zurückzuführen ist. Das Einwachsen der Zellen kann z.B. notwendig sein für den Abbau der Zubereitung oder zur Abgabe bzw. Ablagerung von Substanzen, die z.B. für Gewebsneubildung benötigt werden oder für die Vaskularisation eines Gewebes, das an die Stelle der erfindungsgemäßen Kollagenzubereitung treten soll.

In einer anderen bevorzugten Ausführungsform für die Anwendung auf Wunden oder im Körperinnern wird die erfindungsgemäße Kollagenzubereitung durch Beimischung von Hilfsstoffen wie z.B. Carboxymethylcellulose, Polyacrylsäure, Traganth, Natriumalginat oder Hydroxypropylcellulose so eingestellt, daß sie bioadhäsiv ist, d.h. daß sie durch Grenzflächenkräfte für eine bestimmte Zeit am Oberflächengewebe des Applikationsorts haftet, um so die Verweilzeit am Applikations- bzw. Resorptionsort zu erhöhen.

### Beispiele

### 1. Gewinnung von unlöslichem Kollagen aus Kalbshaut

**1.1**

| | |
|---|---|
| Behandlung mit Ca(OH)₂ 1 % in H₂O | 100 h |
| Behandlung mit NH₄Cl 3 % in H₂O | 1 h |
| Behandlung mit NaOH 5% + Na₂ SO₄ 9,2 % in H₂O | 12 h |
| Behandlung mit Na₂ SO₄ 1 molar in H₂O | 0,5 h |
| Behandlung mit HCl 1 % in H₂O | 1,5 h |
| Behandlung mit H₂O₂ 0,5 % in H₂O | 6 h |

Nach mechanischem Zerkleinern und Dispergieren in H₂O (pH 6,O) weist das unlösliche Kollagen ein mittleres Molekulargewicht von ca. 2.500.000 auf. Die Kollagenkonzentration beträgt 0,75%
**1.2** Wie a), Unterschied:

| | |
|---|---|
| Behandlung mit Ca(OH)₂ 1% in H₂O | 72h |
| Behandlung mit NaOH 9,75 % + Na₂ SO₄ 9,2 % in H₂O | 48h |

Nach mechanischem Zerkleinern und Dispergieren in H₂O (pH 6,0) weist das unlösliche Kollagen ein mittleres Molekulargewicht von ca. 420.000 auf. Die Kollagenkonzentration beträgt 0,75 %.

### 2. Herstellung von wirkstofffreien Lyophilisaten aus Mischungen der Dispersionen nach 1.1 und 1.2 und Bestimmung der Zerfallszeit in Puffergemischen mit unterschiedlichen pH-Werten.

Folgende Abmischungen der Dispersionen nach 1.1 und 1.2 wurden hergestellt:

| Dispersion 1.1 | | Dispersion 1.2 |
|---|---|---|
| A | 100 % | 0 % |
| B | 75 % | 25 % |
| C | 50 % | 50 % |
| D | 25 % | 75 % |
| E | 0 % | 100 % |

Je 24 g der Abmischungen A bis E wurden in Tiefziehteile mit den Abmessungen 6 x 4 x 1,5 cm gefüllt, schockgefroren und innerhalb von 60 Min. auf eine Temperatur von - 50 °C gebracht. Anschließend wurden die Mischungen gefriergetrocknet.

Bei den resultierenden Schwämmen (Abmessungen 6 x 4 x 0,8 cm) wurde unter leichtem Rühren die Zerfallszeit in jeweils 100 ml der folgenden Puffergemische bestimmt:
- pH 3:: Puffergemisch Citronensäure/Natriumchlorid/Natronlauge mit Fungizidzusatz
- pH 5:: Essigsäure (0,1 molar) Natrium-Acetat (0,2 molar)
- pH 6,4:: künstliches Wundsekret (ohne Albumin)
- pH 7,5:: Kaliumhydrogenphospat/NaOH (0,1 molar/0,1 molar)

| **Ergebnisse:** | | | | |
|---|---|---|---|---|
| Mischung | ph der Pufferlösung | | | |
| | 3 | 5 | 6,4 | 7,5 |
| A | 2 h 15 min | > 10 d | > 10 d | > 10 d |
| B | 1 h 10 min | 3 d | 3 d | 2 d |
| C | 1 h | 24 h | 24 h | 6 h |
| D | 20 min | 4 h 10 min | 4 h 30 min | 1 h 45 min |
| E | 2 min | 35 min | 45 min | 15 min |

### 3. Herstellung von Lyophilisaten mit p-Hydroxybenzoesaurepropylester und Bestimmung der Freisetzung

In die Abmischungen A bis E gemäß Beispiel 2 wurde jeweils 0,275 % (bezogen auf die Dispersion) p-Hydroxybenzoesäurepropylester gelöst. Je 10 g der Abmischungen wurden in Tiefziehteile gemäß Beispiel 2 gefüllt, schockgefroren, innerhalb von 60 min auf -50 °C gekühlt und anschließend gefriergetrocknet.

Von den resultierenden Lyophilisaten (Schwammgewichte ca. 135 mg, theor. PHB-Ester-Gehalt 27,5 mg) wurden jeweils ca. 30 mg (theor. PHB-Ester-Gehalt 6,1 mg) in eine paddle-Apparatur gegeben und in 500 ml 0,05 N Natronlauge bei 37 °C mit 45 U/min gerührt. Nach 4 Stunden wurden jeweils 10 ml des Freisetzungsmediums entnommen. Der Wirkstoffgehalt wurde gegen Standard bei 294 nm mit 1 cm Schichtdicke UV-photometrisch bestimmt.

| **Ergebnisse:** | | | | | |
|---|---|---|---|---|---|
| Mischung | A | B | C | D | E |
| freigesetzter Wirkstoff (mg) | 2,1 | 2,7 | 3,4 | 3,8 | * |

| | | | | | |
|---|---|---|---|---|---|
| *konnte nicht bestimmt werden, da Lyophilisat vollständig zerfallen | | | | | |

### 4. Herstellung von Lyophilisaten mit Lidocainhydrochlorid und Bestimmung der Freisetzung

In die Abmischungen A bis E gemäß Beispiel 2 wurden jeweils 0,042 % (bezogen auf die Dispersion) Lidocainhydrochlorid gelöst. Je 24 g der Abmischungen wurden in Tiefziehteile gemäß Beispiel 2 gefüllt, schockgefroren, innerhalb von 60 min auf -50 °C gekühlt und anschliessend gefriergetrocknet.
Die resultierenden Lyophilisate (Schwammgewichte ca. 250 mg, theor. Gehalt Lidocainhydrochlorid 10 mg) wurden jeweils in eine paddle-Apparatur gegeben und mit einem Netz am Boden des Gefäßes fixiert. Es wurden 350 ml Wasser mit einer Temperatur von 37 °C in die Apparatur gegeben und mit 45 U/min gerührt. Nach 24 Stunden wurden Proben des Freisetzungsmediums entnommen. Der Wirkstoffgehalt wurde bei 262,5 nm mit 1 cm Schichtdicke UV-photometrisch mit Auswertung anhand Eichkurve bestimmt.

| **Ergebnisse:** | |
|---|---|
| Mischung | freigesetzter Wirkstoff |
| A | 1,2 mg |
| B | 5,1 mg |
| C | 7,4 mg |
| D | 8,8 mg |
| E | 10,0 mg |

## Patentansprüche

1. Kollagenzubereitung zur gesteuerten Freisetzung von Wirkstoffen, dadurch gekennzeichnet, daß sie Mischungen säureunlöslicher Kollagene mit unterschiedlichen Molekulargewichtsverteilungen aufweist.

2. Kollagenzubereitung nach Anspruch 1, dadurch gekennzeichnet, daß die Kollagenzubereitung unterschiedliche Wirkstoffe enthält.

3. Kollagenzubereitung nach Anspruch 1 oder 2, dadurch gekennzeichnet, daß sie Hilfsstoffe wie Viskositätsregulierungsmittel, Bindemittel, Feuchthaltemittel, Weichmacher, Penetrationsbeschleuniger, Konservierungsmittel, Desinfektionsmittel, pH-Regulatoren, Antioxidantien, Wirkstoffstabilisatoren, Öle, Fette, Wachse Emulsionsstabilisatoren, Duftstoffe, Farbstoffe und/oder inerte Füllstoffe enthält.

4. Kollagenzubereitung nach einem der Ansprüche 1 bis 3, dadurch gekennzeichnet, daß das unlösliche Kollagen telopeptidfreies, natives, unvernetztes Typ-1-Kollagen ist.

5. Kollagenzubereitung nach einem oder mehreren der Ansprüche 1-4, dadurch gekennzeichnet, daß das unlösliche Kollagen ein durch alkalischen Aufschluß aus Kalbshaut gewonnenes Produkt ist.

6. Kollagenzubereitung nach einem oder mehreren der Ansprüche 1 bis 5, dadurch gekennzeichnet, daß die Ausführungsformen der Kollagenzubereitung Pulver, Puder, Mikropartikel, Fasern, Flocken, Schäume, Schwämme, Nadeln, Stäbchen, Tabletten, Gele, Cremes, einschichtige Filme oder Laminate sind.

7. Kollagenzubereitung nach Anspruch 6, dadurch gekennzeichnet, daß die Kollagenzubereitung eine der gewünschten Wirkstofffreisetzungskinetik angemessene Kombination unterschiedlicher Ausführungsformen umfaßt.

8. Kollagenzubereitung nach einem oder mehreren der vorgenannten Ansprüche, dadurch gekennzeichnet, daß sie bioadhäsiv ist.

9. Verfahren zur Herstellung der Kollagenzubereitung nach einem oder mehreren der vorgenannten Ansprüche, dadurch gekennzeichnet, daß sie durch Sprühtrocknung, Gefriertrocknung, Beschichten oder Gießen mit anschließender Trocknung, Phasenseparations- und Koazervationsverfahren, Komprimierung oder Abfüllung in Behältnisse hergestellt wird.

10. Verfahren nach Anspruch 9, dadurch gekennzeichnet, daß die Wirkstoffabgabe durch das Mischungsverhältnis von säureunlöslichen Kollagenen mit unterschiedlichen Molekulargewichtsverteilungen beeinflußt und gesteuert wird.

11. Verfahren nach Anspruch 9 oder 10, dadurch gekennzeichnet, daß die Wirkstoffabgabe durch Auflösung oder Quellung und Erosion der Kollagenzubereitung steuerbar ist.

12. Verfahren nach Anspruch 9 oder 10, dadurch gekennzeichnet, daß die Wirkstofffreisetzung durch die biologische Abbaubarkeit der Kollagenzubereitung steuerbar ist.

13. Verwendung der Kollagenzubereitung nach einem oder mehreren der Ansprüche 1-7 zur Herstellung eines Arzneimittels für die gesteuerte Abgabe von Wirkstoff an Wunden.

14. Verwendung der Kollagenzubereitung nach einem oder mehreren der Ansprüche 1-8 zur Herstellung eines Arzneimittels für die gesteuerte Abgabe von Wirkstoff an intakte Haut.

15. Verwendung der Kollagenzubereitung nach einem oder mehreren der Ansprüche 1-8, zur Herstellung eines Arzneimittels zum Implantieren oder Injizieren von Wirkstoff in einen lebenden Organismus.

## Claims

1. A collagen preparation for the controlled release of active substances characterized in that it has mixtures of acid-insoluble collagens with different molecular weight distributions.

2. The collagen preparation according to claim 1 characterized in that the collagen preparation comprises different active substances.

3. The collagen preparation according to claim 1 or 2 characterized in that it comprises adjuvants, such as viscosity regulators, binders, humectants, softening agents, penetration enhancers, preservatives, disinfectants, pH-regulators, antioxidants, active substance stabilizers, oils, fats, waxes, emulsion stabilizers, odorous substances, dyes, and/or inert fillers.

4. The collagen preparation according to any one of claims 1 to 3 characterized in that the insoluble collagen is telopeptide-free, native, uncross-linked Type-1-collagen.

5. The collagen preparation according to one or several of claims 1-4 characterized in that the insoluble collagen is a product obtained from calfskin by alkaline decomposition.

6. The collagen preparation according to one or several of claims 1 to 5 characterized in that the embodiments of the collagen preparations are powders, dusts, microparticles, fibers, flakes, foams, sponges, needles, small rods, tablets, gels, creams, single-layer films, or laminates.

7. The collagen preparation according to claim 6 characterized in that the collagen preparation comprises a combination of different embodiments appropriate to the desired kinetics of active substance release.

8. The collagen preparation according to one or several of the preceding claims characterized in that it is bioadhesive.

9. A process for the production of the collagen preparation according to one or several of the preceding claims, characterized in that it is manufactured by spray drying, freeze-drying, coating or casting with subsequent drying, phase separation and coacervation processes, compression, or filling into containers.

10. The process according to claim 9 characterized in that the active substance release is influenced and controlled by the mixing ratio of acid-insoluble collagens having different molecular weight distributions.

11. The process according to claim 9 or 10 characterized in that the active substance release can be controlled by dissolution or swelling and erosion of the collagen preparation.

12. The process according to claim 9 or 10 characterized in that the active substance release can be controlled by the biodegradability of the collagen preparation.

13. The use of the collagen preparation according to one or several of claims 1-7 for the production of a pharmaceutical product for the controlled release of active substances to wounds.

14. The use of the collagen preparation according to one or several of claims 1-8 for the production of a pharmaceutical product for the controlled release of active substances to intact skin.

15. The use of the collagen preparation according to one or several of claims 1-8 for the production of a pharmaceutical product for implanting or injecting active substances into a living organism.

## Revendications

1. Préparation de collagène pour la libération contrôlée de principes actifs, caractérisée en ce qu'elle présente des mélanges de collagène insolubles en milieu acide avec différentes distributions des poids moléculaires.

2. Préparation de collagène selon la revendication 1, caractérisée en ce que la préparation de collagène contient différents principes actifs.

3. Préparation de collagène selon la revendication 1 ou 2, caractérisée en ce qu'elle contient des adjuvants, tels que des agents de régulation de la viscosité, des agents liants, des agents humidifiants, des agents plastifiants, des accélérateurs de la pénétration, des agents conservateurs, des agents désinfectants, des régulateurs du pH, des agents antioxydants, des agents stabilisateurs de principes actifs, des huiles, des graisses, des cires, des agents stabilisateurs d'émulsions, des parfums, des colorants et/ou des matières de charge inertes.

4. Préparation de collagène selon l'une des revendications 1 à 3, caractérisée en ce que le collagène insoluble est du collagène de type 1 non-réticulé, natif et exempt de télopeptides.

5. Préparation de collagène selon une plusieurs des revendications 1 à 4, caractérisée en ce que le collagène insoluble est un produit extrait par dissolution alcaline à partir de peaux de veau.

6. Préparation de collagène selon une plusieurs des revendications 1 à 5, caractérisée en ce que les formes de réalisation de la préparation de collagène sont des matières pulvérulentes, des poudres, des microparticules, des fibres, des flocons, des mousses, des éponges, des aiguilles, des baguettes, des comprimés, des gels, des crèmes, des films monocouches ou des stratifiés.

7. Préparation de collagène selon la revendication 6, caractérisée en ce que la préparation de collagène comprend une combinaison de différentes formes de réalisation, appropriée pour la cinétique souhaitée de libération de principes actifs.

8. Préparation de collagène selon une plusieurs des revendications précédentes, caractérisée en ce qu'elle est bioadhésive.

9. Procédé de préparation de la préparation de collagène selon une ou plusieurs des revendications précédentes, caractérisé en ce qu'elle est fabriquée par séchage par pulvérisation, par lyophilisation, par enduction ou coulée suivie d'un séchage, par un procédé de séparation de phases et de coacervation, par compression ou par conditionnement dans des récipients.

10. Procédé selon la revendication 9, caractérisé en ce que la libération de principes actifs est influencée et contrôlée par le rapport de mélange des collagènes insolubles en milieu acide avec différentes distributions de poids moléculaires.

11. Procédé selon la revendication 9 ou 10, caractérisé en ce que la libération de principes actifs peut être contrôlée par la dissolution ou le gonflement et l'érosion de la préparation de collagène.

12. Procédé selon la revendication 9 ou 10, caractérisé en ce que la libération de principes actifs peut être contrôlée par la biodégradabilité de la préparation de collagène.

13. Utilisation de la préparation de collagène selon une ou plusieurs des revendications 1 à 7, pour la préparation d'un médicament pour la libération contrôlée de principes actifs à des blessures.

14. Utilisation de la préparation de collagène selon une ou plusieurs des revendications 1 à 8, pour la préparation d'un médicament pour la libération contrôlée de principes actifs à la peau intacte.

15. Utilisation de la préparation de collagène selon une ou plusieurs des revendications 1 à 8, pour la préparation d'un médicament en vue de l'implantation ou de l'injection de principes actifs dans un organisme vivant.
